# EUROPEAN PATENT APPLICATION

(11) **EP 2 708 192 A1**
(43) Date of publication of application: **19.03.2014**
(21) Application number: 13181164.8
(22) Date of filing: 21.08.2013
(51) Int. Cl.: A61B 17/16

(54) **Bone graft shaper**

(30) Priority: 14.09.2012 US 201213616848
(71) Applicant: DePuy Synthes Products, LLC, Raynham, MA 02767-0350 (US)
(72) Inventor: Klotz, Conrad, Warsaw, IN Indiana 46581 (US); Chavarria, Jason M, Warsaw, IN Indiana 46581 (US)
(74) Representative: Belcher, Simon James

(57) **Abstract**

An instrument (40) for use in preparing a graft includes at least one cutting portion (46) configured to resect a graft section from the graft. The instrument also includes a drive portion (42) operably coupled to the cutting portion and configured to receive a rotational force. At least one reaming portion (44) is positioned proximally to the cutting portion, the reaming portion is configured to ream a surface of the graft. The reaming portion and the cutting portion are positioned with respect to each other such that when the instrument is positioned against the graft and the rotational force is applied to the drive portion, the cutting section rotationally forms the graft section from the graft and the reaming portion rotationally reams a proximal section of the graft.

## Description

The present invention relates to an instrument for shaping a bone graft. It can be used during a surgical procedure to replace part or all of a patient's shoulder joint.

In the shoulder, a person's glenoid fossa may become worn, causing severe shoulder pain and limiting the range of motion of the patient's shoulder joint. Shoulder arthroplasty may be performed to alleviate such pain and increase the patient's range of motion. Arthroplasty is the surgical replacement of one or more bone structures of a joint with one or more prostheses.

Shoulder arthroplasty often involves replacement of the glenoid fossa of the scapula with a prosthetic glenoid component. The conventional glenoid component typically provides a generally laterally or outwardly facing generally concave bearing surface against which a prosthetic humeral head (or, alternatively, the spared natural humeral head in the case of a glenoid hemi-arthroplasty) may bear during operation of the joint. The conventional glenoid component typically also includes a generally medially or inwardly projecting stem for fixing the glenoid component in a cavity constructed by suitably resecting the glenoid fossa and suitably resecting cancellous bone from the glenoid vault.

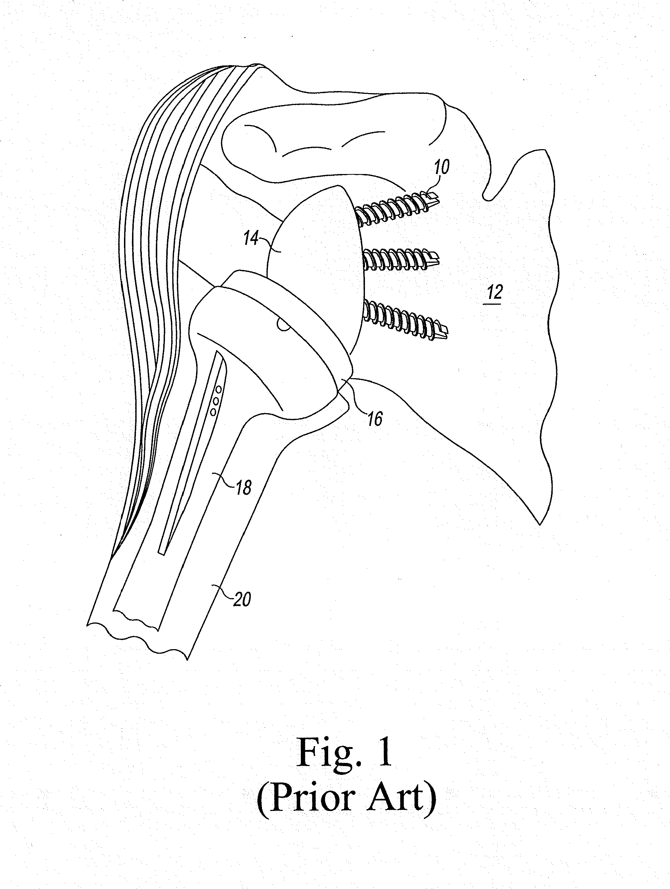

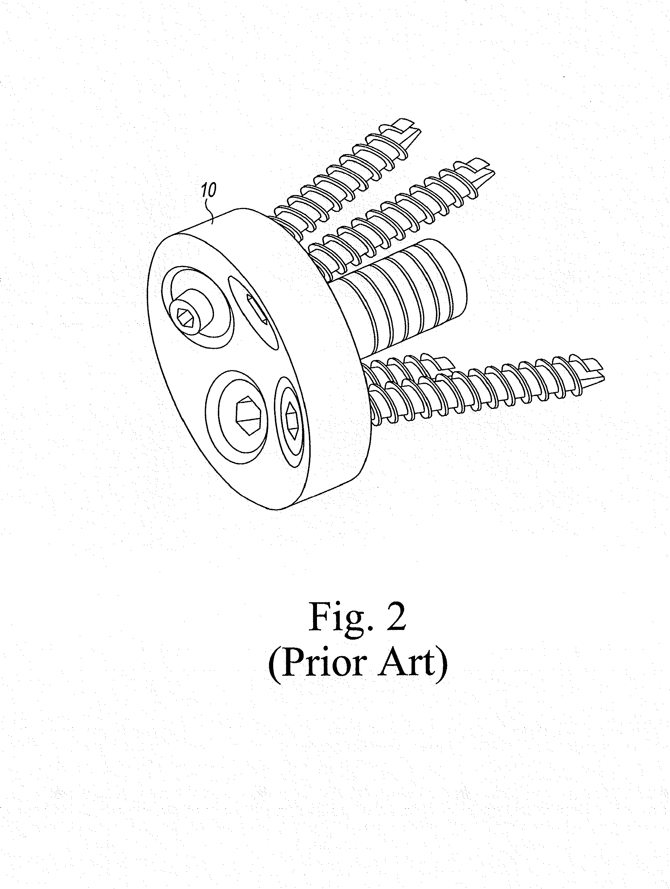

However, in patients suffering from Cuff Tear Arthropathy (CTA), a standard shoulder replacement is not an option. In such cases, a reverse shoulder is often used. A reverse shoulder, as shown in FIGS. 1 and 2, includes inserting a metaglene 10 into the glenoid fossa 12. A glenosphere 14 is coupled to the metaglene. The glenosphere 14 articulates against a cup 16 that is attached to a stem 18, inserted into a humerus 20. These types of procedures are generically referred to as reverse shoulder arthroplasty.

In some forms of cuff tear arthropathy, the glenoid vault may be severely eroded. In such instances, the metaglene 10 cannot properly fit on the glenoid fossa 12. When dealing with those types of situations, surgeons may use bone graft to fill in the space in the glenoid vault. In some instances, the bone graft may be formed from the resected humeral head. Currently, surgeons may use allograft or bone resected from other parts of the body such as the resected humeral head or portions of the femur. In these types of resections, the surgeon must resect the bone graft (or allograft) from the humeral head using tools created for other uses. This results in poorly shaped bone grafts and requires a lot of extra time in the operating room.

The present invention provides an instrument for use in resecting and shaping a properly shaped bone graft or allograft.

The present invention provides an instrument which includes at least one cutting portion configured to resect a graft section from the graft. The instrument also includes a drive portion operably coupled to the cutting portion and configured to receive a rotational force. At least one reaming portion is positioned proximally to the cutting portion, the reaming portion is configured to ream a surface of the graft. The reaming portion and the cutting portion are positioned with respect to each other such that when the instrument is positioned against the graft and the rotational force is applied to the drive portion, the cutting section rotationally forms the graft section from the graft and the reaming portion rotationally reams a proximal section of the graft.

The invention also provides a kit which includes a power extension adapted to couple to a power tool and having a power transfer portion at one end. The kit also includes an instrument having at least one cutting portion configured to resect a graft section from the graft. The instrument further includes a drive portion operably coupled to the cutting portion and configured to receive a rotational force. At least one reaming portion is positioned proximally to the cutting portion, the reaming portion configured to ream a surface of the graft. A drilling portion is also part of the instrument and is configured to rotationally create a bore in the graft. The drilling portion is positioned distally from the reaming portion and is operably coupled to the drive portion. The reaming portion and cutting portion are positioned with respect to each other such that when the instrument is positioned against the graft and the rotational force is applied, the cutting portion rotationally forms the graft section and the reaming portion rotationally reams a proximal section of the graft section. Also, the drive portion is adapted to be coupled to the power transfer portion of the power extension.

The invention also provides a method for preparing a graft section from a sample. An instrument is used, the instrument having at least one cutting portion configured to resect the graft section from the bone sample, at least one reaming portion positioned proximally to the cutting portion, the reaming portion configured to ream a surface of the graft section, and a drilling portion configured to rotationally create a bore in the graft, the drilling portion being positioned distally from the reaming portion. The sample is obtained and instrument is positioned adjacent the sample. The cutting portion cuts a section of the sample, while the proximal section of the sample is reamed and a bore is drilled in the sample.

The invention is described below by way of example with reference to the accompanying drawings, in which:
FIG. 1 is a partial cut-away view of a reverse shoulder implant as known in the prior art.
FIG. 2 is a perspective view of a metaglene as known in the prior art.
FIG. 3 is a partial cut-away view of an instrument for shaping a bone graft.
FIG. 4 is a side view of the instrument of FIG. 3.
FIG. 5 is a bottom view of the instrument of FIG. 3.
FIG. 6 is a partial cut-away view of the instrument of FIG. 3.
FIG. 7 is a side view of a power extension which can be used with the instrument in a kit.
FIG. 8 is a combination of the power extension of FIG. 7 and the instrument of FIG. 3.
FIG. 9 is another view of the combination of FIG. 8.
FIG. 10 is a flow chart illustrating a method of using the instrument.
FIG. 11 is a plan view of a resected humeral head.
FIG. 12 is a plan view of an assembled instrument, power extension and guide pin.
FIG. 13 is a plan view of the assembled instrument of FIG. 12 adjacent a humeral head.
FIG. 14 is a plan view of the assembled instrument of FIG. 12 cutting through the humeral head.
FIG. 15 is a plan view of the humeral head after the instrument has been used.
FIG. 16 is a plan view of the resulting resected bone graft.
FIG. 17 is a perspective view of the resulting resected bone graft attached to a metaglene.
FIG. 18 is a perspective view of the resulting resected bone graft of FIG. 17 attached to a metaglene and a head.
FIG. 19 shows an instrument to aid in reaming a resected bone graft.
FIG. 20 is a perspective view of the instrument of FIG. 19.
FIG. 21 is another perspective view of the instrument of FIG. 20.

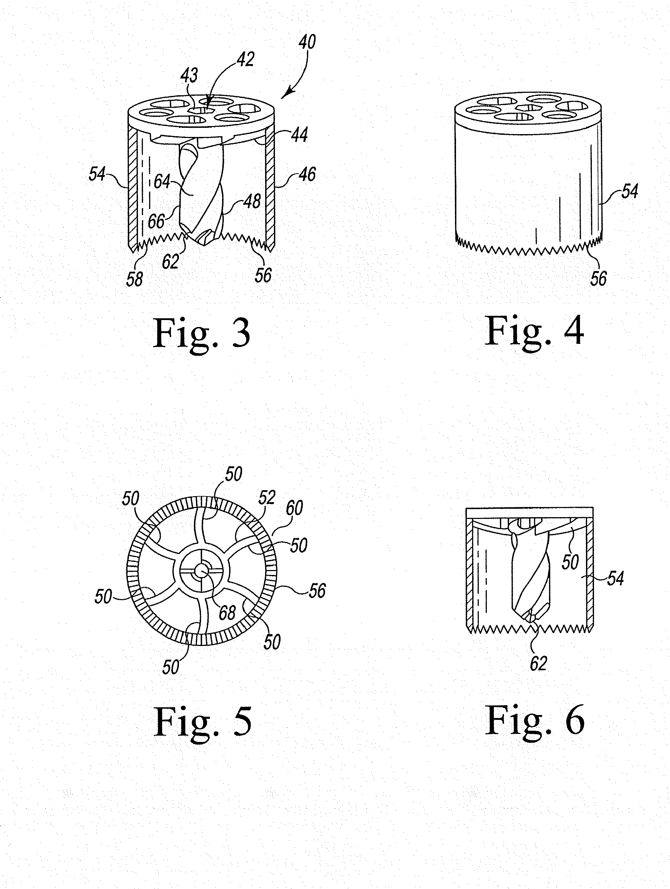

Referring to the drawings, FIGS. 3 to 6 show an instrument 40 for preparing a bone graft from a bone sample. In this document, the term "bone sample" is meant to include any type of resected bone or allograft. The instrument includes a drive portion 42, a reaming portion 44, a cutting portion 46, and a drill or boring portion 48. The drive portion 42 in this embodiment is a hexagonally shaped bore 43 defined in the reaming portion 44. The instrument includes a body portion 49 that defines the hexagonally shaped bore 43. The reaming portion 44, cutting portion 46 and drill portion 48 all extend distally from the body portion 49.

A number of reaming fins 50 extend from the reaming portion 44 toward the drill portion 48. The reaming fins 50 curve proximally and outwardly from the lower central portion of the reaming portion 44 to the outer periphery of the cutting portion 46. The reaming fins 50 include an arcuate leading edge 52.

The cutting portion 46 includes a smooth cylindrical wall 54 surrounding the reaming portion 44. In the instrument shown in FIGS. 3 to 6, the cylindrical wall 54 is smooth and includes an edge 56 at the distal end of the device 40. The edge 56 includes a plurality of cutting teeth 58. In the illustrated embodiment, only the edge 56 includes cutting teeth 58. It is envisaged that the cylindrical wall 54 might not be smooth. For example, it may include cutting teeth 58 on the outside of the cylindrical wall 54. Cutting teeth 58 might be located on the interior of the cylindrical wall 54.

The drilling portion 48, which may include a drill bit, extends away from the reaming portion 44 to a distal tip 62. Two flutes 64 and 66 extend helically about the drilling portion 48 between the reaming portion 44 and the distal tip 62. A guide bore 68 extends from the distal tip 62 to the drive portion 42. In the instrument shown in the drawings, the distal tip 62 does not extend past the edge 56 of the cutting portion 46. It is envisaged that the distal tip 62 might extend past the edge 56 of the cutting portion 46.

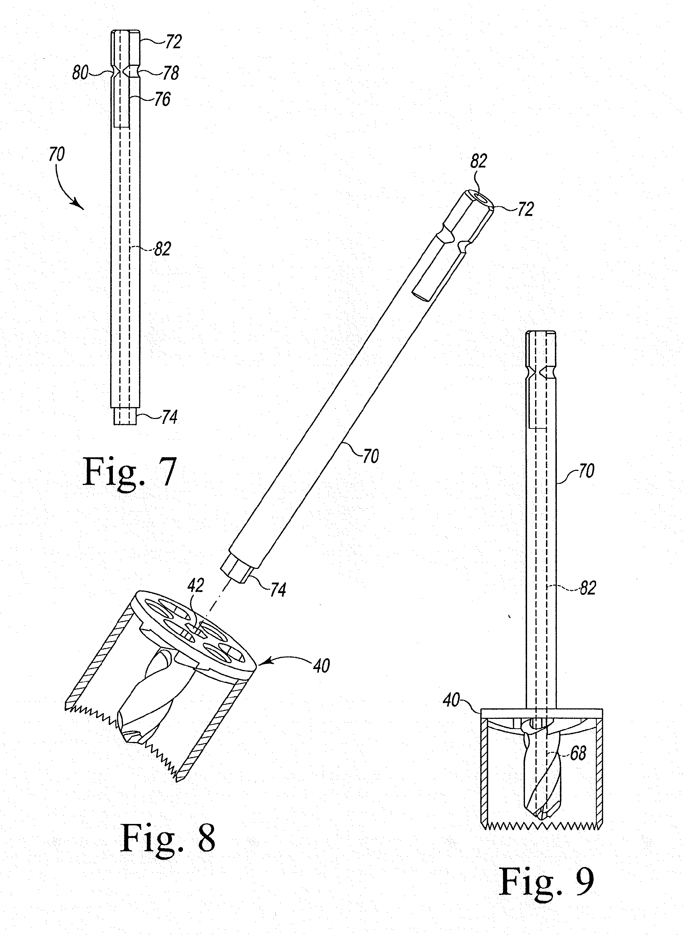

As discussed in further detail below, a kit may include one or more instruments 40 along with various instrumentation to facilitate use of the instrument 40. By way of example, FIG. 7 depicts a power extension 70 that may be included in the kit. The power extension 70 includes a power receiving portion 72 and a power transfer portion 74. The power receiving portion 72 is sized and configured to couple with a power tool and includes a pair of opposing power receiving flats 76 and a pair of coupling grooves 78 and 80 which extend about the power receiving portion 72 between the power receiving flats 76.

The power transfer portion 74 is shaped to be complimentary to the drive portion 42. In the embodiment of FIGS. 7 and 8, the power transfer portion 74 is thus a hexagonally shaped protrusion sized to fit within the drive portion 42. A guide bore 82 extends from the distal tip of the power transfer portion 74 to the proximal end of the power receiving portion 72.

To couple the instrument 40 with the power extension 70, the power transfer portion 74 is aligned with the drive portion 42 as shown in FIG. 8. The instrument 40 with the power extension 70 are then moved toward each other such that the power transfer portion 74 enters into the drive portion 42 resulting in the configuration of FIG. 9. In FIG. 9, the guide bore 68 of the instrument 40 is aligned with the guide bore 82 of the power extension 70.

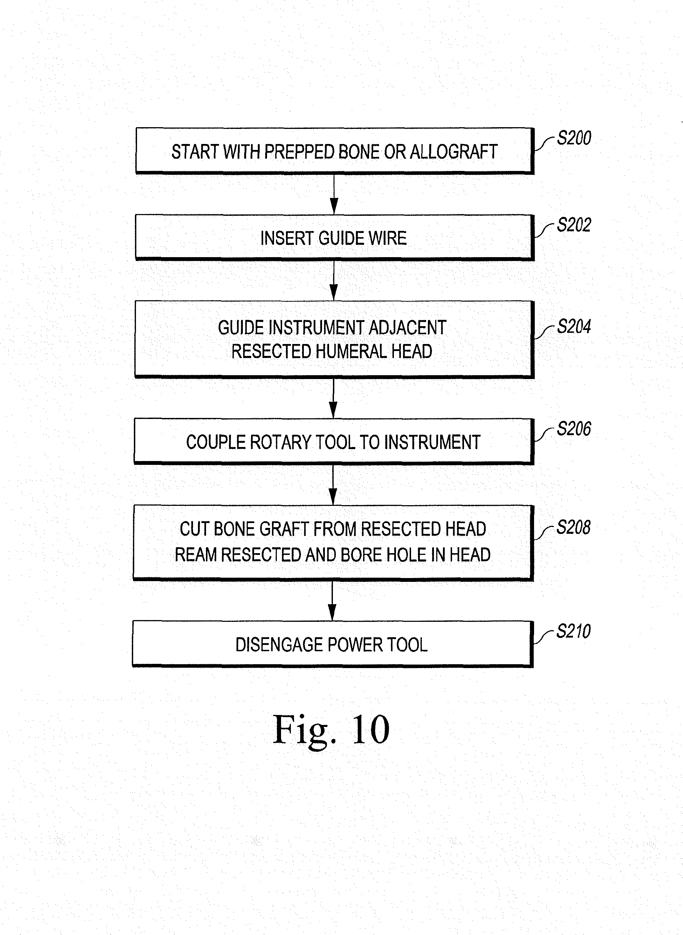

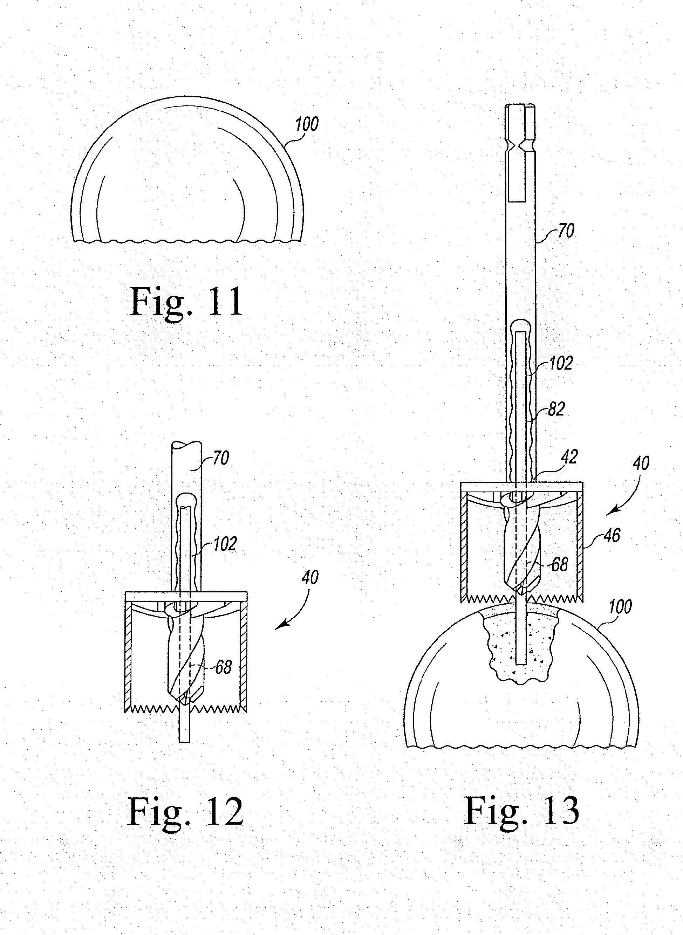

A kit including the instrument 40 and the power extension 70 may be used in preparing a bone graft from a resected humeral shoulder 100 (FIG. 11) in accordance with the procedure shown in FIG. 10. Initially, at step s200, the surgeon or other hospital staff begins the procedure by preparing the bone sample, which may be allograft or bone graft. A humeral head may be used. However, in other applications, allograft or bone grafts from other sites of the body may be used. When a humeral head 100 (FIG. 11) is used, at step s200 the humeral head is resected from the humerus. A guide wire 102 (FIG. 13), which may be provided in a kit along with other instrumentation used in the procedure, may be positioned on the humeral head 100 at step s202. A guide wire need not be used and this step may be skipped. When a guide wire is used, the positioning of the guide wire may be computer aided. The guide wire 102 is put into place such that the instrument 40 will be able to get a complete wedge of bone material.

At step s204, the assembled power extension 70 and instrument 40 are slid over the guide wire. The guide wire 102 extends through the guide bore 68 of the instrument 40 and the guide bore 82 of the power extension 70 as shown in FIG. 13. The guide wire 102 thus guides the power extension 70 and the instrument 40. It is envisaged that the instrument 40 may be inserted onto the guide wire 102 prior to the power extension 70 being assembled to the instrument 40. In that instance, the instrument 40 is first inserted over the guide wire 102 and then the power extension 70 is inserted over the guide wire 102. Some rotation of the power extension 70 may be required to align the power transfer portion 74 with the drive portion 42 of the instrument 40 to allow coupling of the power extension to the instrument.

A rotary tool (not shown) is then coupled to the instrument 40 at step s206. Optionally, a rotary tool may be directly coupled to the instrument 40. In this example, the power extension 70 is coupled to the instrument 40 as described above. Thus, the rotary tool is coupled to the power receiving portion 72 of the power extension 70 so as to be indirectly coupled to the instrument 40.

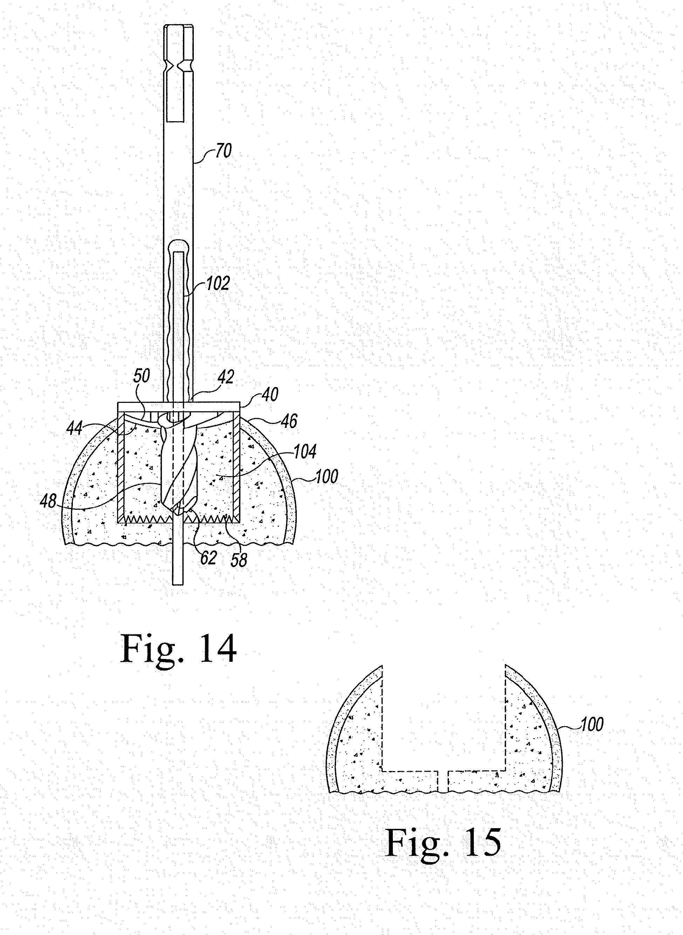

Power is then applied to the rotary tool causing the rotary tool to rotate the power extension 70. Rotary force is transferred to the drive portion 42 of the instrument 40 through the power transfer portion 74. As the instrument 40 initially rotates about the guide wire 102, the reaming portion 44, the cutting portion 46, and the drilling portion 48 all contact the resected humeral head 100 and begins to cut a bone graft 104 from the head at step s208. The cutting, drilling and reaming all occur as a single step, reducing time in the operating room. As the cutting portion 46 cuts the bone graft 104, the drilling portion 48 then engages the bone graft 104 and begins boring a hole over the guide wire 102. As the bone graft 104 is created and a hole is formed in the bone graft 104 by the drilling portion 48, the instrument 40 is guided by the guide wire 102 such that the reaming fins 50 come into contact with a proximal portion of the bone graft 104 as depicted in FIG. 14. Continued rotation of the instrument 40 with the rotary tool thus causes simultaneous reaming of the bone graft 104 with the reaming fins 50 and boring of the bone graft 104 with the drilling portion 48.

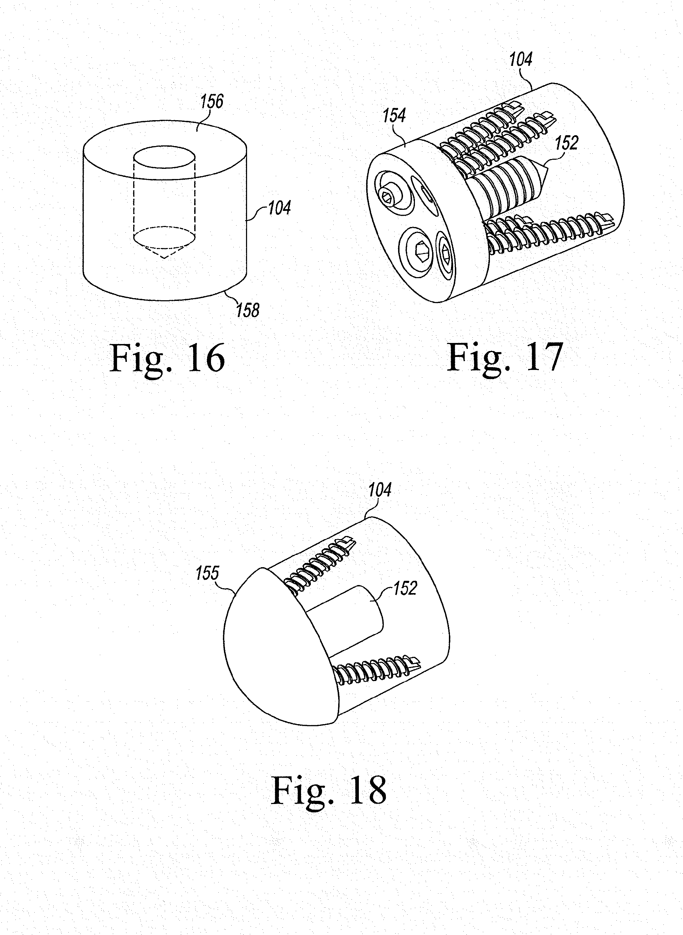

The power tool is de-energized and disconnected at step s210. At step s208, a cutting guide or other tool may be used to disengage the bone graft 104 from the humeral head 100, leaving the humeral head 100 as shown in FIG. 15. The completely removed bone graft 104 is shown in FIG. 16.

The size of the drilling portion 48, both in length and diameter, is selected to be complimentary to the size of a centre peg 152 of a metaglene component 154 (FIG. 17). Thus, upon completion of the reaming, the bore formed by the drilling section is sized to receive the centre peg 152.

The bone graft 104, as shown in FIG. 16, is completely cut from the humeral head. FIG. 17 shows the bone graft 104 as it is coupled to the metaglene component 154. A head 155 is then placed on the metaglene 154 as shown in FIG. 18 and the combination of the bone graft 104, the metaglene component 154, and head 155 are then fit into the glenoid. The rest of the reverse shoulder may then be assembled as is known in the art.

The above method describes using the instrument 40 to prepare a portion of bone graft (or allograft) such that the reaming portion 44 is configured to prepare a surface that will mate with the back side of the metaglene component 154. It is also envisaged that the reaming portion 44 may be used to prepare a surface that will mate with the glenoid. In other words, the bone graft 104 will have two opposing sides 156, 158 as shown in FIG. 16. One of the opposing sides 156 is configured to abut the metaglene component 154 and the other of the opposing sides 158 is configured to abut the glenoid. The reaming portion 44 of the instrument 40 may be configured to prepare either of the two opposing sides 156, 158. The other side must be prepared using a different instrument, such as a cutting guide or reamer.

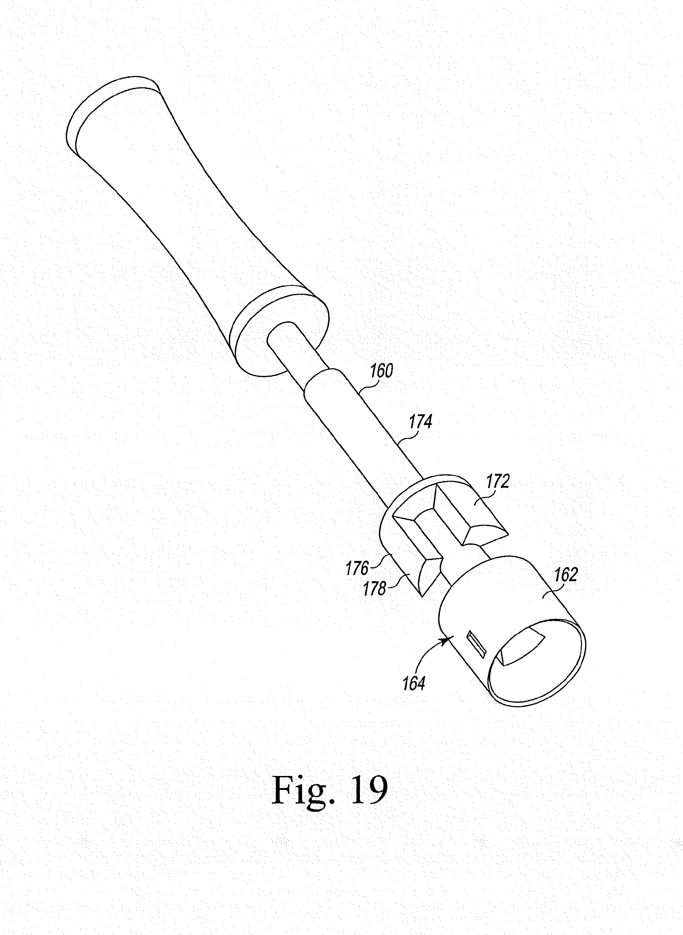

FIG. 19 shows an instrument 160 to assist the surgeon when performing the reaming step. The instrument 160 shown in the drawing is a holder. As stated above, the instrument 40 is used to prepare one side (in this device, the side 158 that will abut the glenoid, although it may be either side). After the bone graft 104 is removed from the original head 100, the bone graft 104 is inserted into the holder 160. The holder 160 includes a cylinder 162 sized and shaped to hold the prepared bone graft 104. The cylinder 162 may include slits 164, which will be described in more detail below.

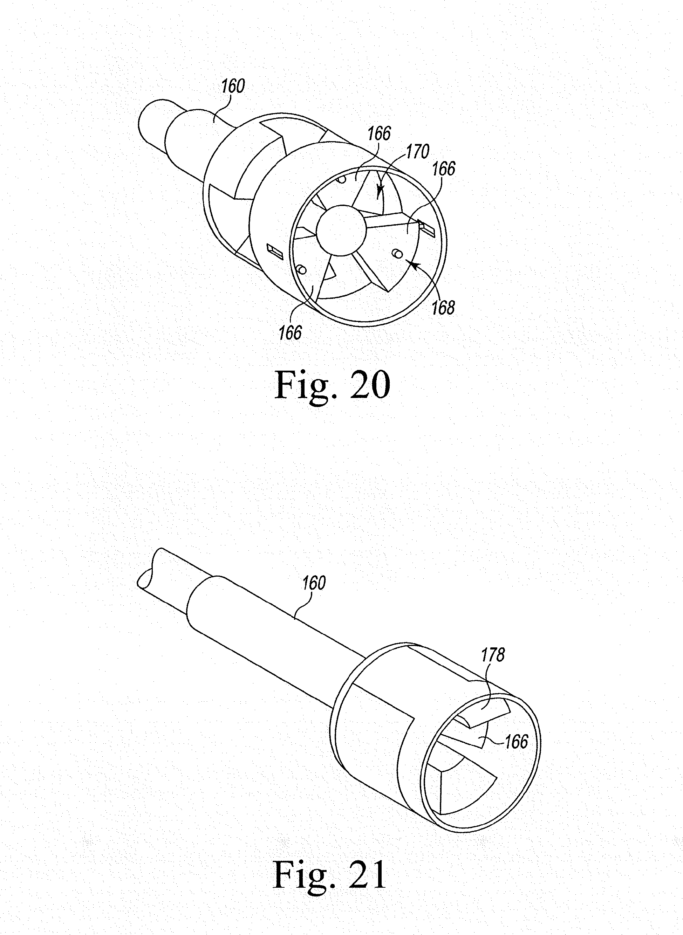

As shown in FIG. 20, the holder 160 includes flanges 166 that extend from the inside of the cylindrical wall 162. The flanges 166 include spikes 168. The spikes 168 extend out from the flanges 166 and are used to grip the bone graft 104 into place. The flanges 166 will define openings 170 between them. The holder 160 also includes a punch 172, having a sliding portion 174 and an extending portion 176 extending distally from the sliding portion. In this instrument, the extending portion 176 includes a plurality of extending walls 178 that fit within the openings 170 of the cylinder 162.

Once the bone graft 104 is securely in place, a reamer (not shown) may be used to ream the other side 156 of the bone graft 104. The reamer is used to prepare the other side 156 of the bone graft 104 to abut the metaglene component 154 (FIG. 17). The slits 164 may be used as a visual aid in the reaming process. In other words, the user could view the reamer through the slits 164 and ensure that the reamer is reaming to the correct depth. After the other side 156 of the bone graft 104 is prepared, the punch 172 is slid down the holder 160, such that the extending walls 178 slide through the openings 170 (shown in FIG. 21) and push the bone graft 104 out of the holder 160. A completely formed bone graft 104 is now ready for use.

## Claims

1. An instrument for use in preparing a graft, the instrument comprising:
at least one cutting portion configured to resect a graft section from the graft,
a drive portion operably coupled to the cutting portion and configured to receive a rotational force,
at least one reaming portion positioned proximally to the cutting portion, the reaming portion configured to ream a surface of the graft section,
in which the reaming portion and the cutting portion are positioned with respect to each other such that when the instrument is positioned against the graft and the rotational force is applied to the drive portion, the cutting section rotationally forms the graft section from the graft and the reaming portion rotationally reams a proximal section of the graft.

2. The instrument of claim 1, which includes a drilling portion configured to rotationally create a bore in the graft, in which the drilling portion is positioned distally from the reaming portion and the drilling portion being operably coupled to the drive portion.

3. The instrument of claim 2 which includes the body portion, in which the body portion defines the drive portion and the reaming portion extends distally from the body portion and the drilling portion extends distally from the body portion.

4. The instrument of claim 2, the instrument which includes a guide bore extending from a distal tip of the drilling portion to the drive portion.

5. The instrument of claim 2, in which the drilling portion includes two flutes extending helically about the drilling portion.

6. The instrument of claim 1, in which the reaming portion comprises:
a plurality of reaming fins extending distally from the body portion, each of the plurality of reaming fins defining an arcuate leading edge.

7. The instrument of claim 1, in which the cutting portion includes a cylindrical wall and a distal edge, the distal edge including a plurality of cutting teeth.

8. The instrument of claim 6, in which the cylindrical wall of the cutting portion is smooth.

9. An kit for use in preparing a graft, comprising:
a power extension adapted to couple to a power tool and having a power transfer portion at one end, and
an instrument as defined in any one of claims 1 to 8,
in which the drive portion is adapted to be coupled to the power transfer portion of the power extension.

10. The kit of claim 9, the instrument which includes a guide bore extending from a distal tip of the drilling portion to the drive portion and the power extension including a guide bore extending throughout the length of the power extension.

11. The kit of claim 9, which includes a graft holder, the graft holder including a cylinder sized and shaped to hold the resected graft.

12. The kit of claim 9, which includes a metaglene implant component.

13. A method for preparing a graft section from a sample, the method comprising:
using an instrument having at least one cutting portion configured to resect the graft section from the bone sample, at least one reaming portion positioned proximally to the cutting portion, the reaming portion configured to ream a surface of the graft section, and a drilling portion configured to rotationally create a bore in the graft, the drilling portion being positioned distally from the reaming portion,
obtaining the sample,
positioning the instrument adjacent the sample,
cutting, via the cutting portion, a section of the sample,
reaming, via the reaming portion, a proximal section of the sample, and
drilling, via the drilling portion, a bore in the sample.

14. The method of claim 13, in which the steps of cutting, reaming, and drilling are accomplished in a single step.

15. The method of claim 13, which includes using a metaglene implant component and attaching the metaglene implant component to the bone graft section prepared from the bone graft, and in which optionally the reaming step comprises reaming the proximal section of the bone section to match a back side of the metaglene implant component.
